# EUROPÄISCHE PATENTSCHRIFT

(11) **EP 2 170 197 B1**
(45) Veröffentlichungstag und Bekanntmachung des Hinweises auf die Patenterteilung: **06.04.2011**
(21) Anmeldenummer: 08773560.1
(22) Anmeldetag: 20.06.2008
(51) Int. Cl.: A61B 18/02, A61B 10/02

(54) **KRYOBIOPSIESONDE**
CRYOBIOPSY PROBE
SONDE DE CRYOBIOPSIE

(30) Priorität: 26.06.2007 DE 102007029387; 04.06.2008 DE 102008026635
(43) Veröffentlichungstag der Anmeldung: 07.04.2010
(73) Patentinhaber: Erbe Elektromedizin GmbH, 72072 Tübingen (DE)
(72) Erfinder: FISCHER, Klaus, 72202 Nagold (DE); SZYRACH, Mara, 72070 Tübingen (DE); SCHÄLLER, Daniel, 72070 Tübingen (DE); VOIGTLÄNDER, Matthias, 72810 Gomaringen (DE); SIGLE, Irina, 72116 Mössingen (DE)
(74) Vertreter: Bohnenberger, Johannes
(86) Internationale Anmeldenummer: PCT/EP2008/005010
(87) Internationale Veröffentlichungsnummer: WO 2009/000477

(56) Entgegenhaltungen:
- EP-A- 0 119 176
- WO-A-2007/025106
- WO-A-2008/074422

## Beschreibung

Die Erfindung betrifft ein kryochirurgisches Instrument zur Gewinnung einer Gewebeprobe.

Die Kryobiopsie ist ein spezielles Verfahren im Bereich der Endoskopie, um Gewebeproben aus dem Körper eines Patienten zu entnehmen und somit die Diagnose über ein eventuelles Krankheitsbild nicht nur zu erleichtern, sondern auch zu verbessern.

Der Entnahmeprozess verläuft wie folgt. Eine Kryosonde wird mit leichtem Druck auf das zu untersuchende Gewebe aufgelegt. Durch ein starkes Abkühlen des Sondenkopfs wird das Gewebe an dieser Stelle an die Sonde angefroren. Nach einer vorgegebenen Zeit wird das angefrorene Gewebe durch mechanischen Zug an der Sonde extrahiert.

Das schnelle und starke Abkühlen des Sondenkopfs (ca. -50° C) wird durch den Joule-Thomson-Effekt erreicht. Hierbei macht man es sich zu Nutzen, dass sich Gas stark abkühlt, wenn es unter hohem Druck durch eine kleine Düse in einen großen Raum expandieren kann. Diese Expansion findet im Sondenkopf statt. Durch die Expansion des Gases entsteht Energie in Form von Kälte.

Zur Durchführung einer Kryobiopsie sind unterschiedliche Geräte bekannt. Ein kryochirurgisches Gerät geht aus der US 2003/0195436 A1 hervor. Dieses Instrument ist besonders zum Sichten und "Entkernen" von Tumoren geeignet. Das Instrument umfasst einen Handgriff, der mit einer Sonde verbunden ist. Die Sonde weist an ihrem distalen Ende einen Sondenkopf auf, der eine lanzenförmige Ausgestaltung hat. Der Sondenkopf lässt sich, wie beschrieben, stark abkühlen. Des Weiteren weist das Instrument eine Entnahmekanüle auf. Zur Entnahme wird das Gewebe mit der Lanze bzw. dem Sondenkopf aufgespießt und an diesem festgeeist. Durch ein Vorschieben der Entnahmekanüle wird ein Teil des festgeeisten Gewebes aus dem Tumor ausgeschnitten. Durch ein Abziehen des Instruments kann die ausgeschnittene Gewebeprobe entnommen werden. Um den Ausschneidevorgang zu erleichtern, weist die US 2003/0195436 A1 eine Beschleunigungsvorrichtung auf, die durch eine Feder angetrieben wird und die Kanüle mit einer vorbestimmten Kraft in das Gewebe treibt.

Zur Entnahme von Gewebeproben an Gewebeoberflächen ist dieses kryochirurgische Instrument nicht geeignet. Des Weiteren lassen sich hiermit nur relativ große Mengen von Gewebe entnehmen. Das entsprechende Verfahren verursacht eine tiefe Verletzung des zu behandelnden Gewebes.

Ein weiteres kryochirurgisches Instrument ist durch die EP 0 119 176 A bekannt, von der der Anspruch 1 abgeleitet wird.

Ausgehend von diesem Stand der Technik ist es Aufgabe der vorliegenden Erfindung, ein verbessertes kryochirurgisches Instrument bereit zu stellen. Das Instrument soll insbesondere dafür geeignet sein, Gewebeproben von Gewebewänden, insbesondere Schleimhäuten zu entnehmen. Dabei soll es zu einer möglichst geringen Verletzung des umliegenden Gewebes kommen und die Probe an sich sollte möglichst gut erhalten bleiben. Der Entnahmevorgang sollte einfach und effizient sein.

Diese Aufgabe wird durch das kryochirurgische Instrument gemäß dem Anspruch 1 gelöst.

Insbesondere wird die Aufgabe durch ein kryochirurgisches Instrument zur Gewinnung einer Gewebeprobe gelöst, wobei das Instrument Folgendes umfasst:
- eine Sonde mit einem Sondenkopf, der zur Fixierung eines Abschnitts einer Gewebewand durch eine Kühleinrichtung kühlbar ist;
- einen Stützschlauch mit einem Distalende, wobei die Sonde im Stützschlauch relativ zu diesem beweglich gelagert ist; und
- eine Beschleunigungsvorrichtung zur Beschleunigung des Stützschlauchs mit einer vorbestimmten Beschleunigungskraft relativ zur Sonde, wobei die Beschleunigungskraft derart gerichtet ist, dass die Sonde aus einer ausgefahrenen Position, in der der Sondenkopf das Distalende des Stützschlauchs überragt, in das Innere des Stützschlauchs zurückgezogen wird, wobei das Distalende des Stützschlauchs derart ausgestaltet ist, dass es bei einer Kontaktierung der Gewebewand eine Abrisskraft so induziert, dass durch die Sonde ein Abschnitt der Gewebewand herausgerissen wird.

Ein zentraler Gedanke der vorliegenden Erfindung besteht also darin, dass die Gewebeprobe bzw. das Biopsat nicht aus der Gewebewand herausgeschnitten, sondern durch eine wohldefinierte Kraft herausgerissen wird. Erfindungsgemäß wird hierfür der Sondenkopf an der Gewebewand festgeeist und dann mit einer Abrisskraft in den Stützschlauch eingezogen. Um den Abrissvorgang erfolgreich durchführen zu können, ist es notwendig, einen geeigneten Impuls auf das angeeiste Gewebe auszuüben. Für den die Behandlung durchführenden Arzt ist es problematisch, den geeigneten Impuls mittels des Sondenkopfs auszuüben. Hierfür stellt die vorliegende Erfindung die Beschleunigungsvorrichtung bereit. Diese beschleunigt den Stützschlauch relativ zur Sonde. Durch das Auftreffen des beschleunigten Stützschlauchs auf die Gewebewand wird eine Abrisskraft freigesetzt, mittels derer der Sondenkopf einen Bereich der Gewebewand herausreißt. Die Abrisskraft wirkt im Wesentlichen in entgegen gesetzter Richtung wie die Beschleunigungskraft, die auf den Stützschlauch ausgeübt wird. Durch das Ausreißen kann eine vorteilhafte Abtrennung des Gewebes erzielt werden, bei der natürliche Zellgrenzen berücksichtigt werden.

Der Sondenkopf kann einen Planarabschnitt zur Kontaktierung mit dem Gewebe umfassen. Dieser Abschnitt verläuft im Wesentlichen senkrecht zur Längsachse der Sonde und sollte derart ausgestaltet sein, dass eine möglichst großflächige Kontaktierung mit der Gewebewand möglich ist. Der Planarabschnitt kann entweder eben sein oder eine geeignete Oberflächenstruktur aufweisen. Beispielsweise könnte der Sondenkopf leicht abgerundet sein. Entscheidend ist, dass bei einem Andrücken des Sondenkopfs an das Gewebe eine großflächige Kontaktfläche hergestellt wird, die durch das Kühlen mit der Kühleinrichtung an dem Planarabschnitt festgeeist wird. Somit lässt sich die Abrisskraft problemlos auf das Gewebe übertragen.

Die Beschleunigungsvorrichtung kann eine pneumatische und/oder eine elektromagnetische und/oder eine mechanische und/oder eine pyrotechnische Beschleunigungsvorrichtung umfassen. Somit kann eine vordefinierte Beschleunigungskraft bereitgestellt werden. Die definierte Abrisskraft führt bei dem Auslösen von Biopsat zu vorteilhaften Ergebnissen. Der Arzt muss den geeigneten Impuls nicht manuell festlegen. Bei jeder Biopsie wird die gleiche Menge an Kraft aufgebracht.

Die Beschleunigungsvorrichtung kann eine Feder umfassen. Die Feder kann vor dem Eingriff gespannt werden und speichert eine definierte Menge an potentieller Energie.

Die Beschleunigungsvorrichtung kann ein Verbindungsstück umfassen, das mit dem Stützschlauch in Wirkverbindung steht und mittelbar oder unmittelbar an die Sonde angekoppelt ist, wobei das Verbindungsstück ein Spannelement umfasst, das eine vorbestimmte Menge an potentieller Energie speichert. Vorzugsweise wird die oben beschriebene Feder im Inneren des Verbindungsstückes gelagert.

Die Beschleunigungsvorrichtung kann eine Auslösevorrichtung umfassen, die die potentielle Energie freisetzt. Somit kann der Entnahmevorgang per Knopfdruck durchgeführt werden.

Das kryochirurgische Instrument kann ein Griffstück umfassen, wobei die Sonde und/oder der Stützschlauch abnehmbar an dem Griffstück angekoppelt ist. Somit ist es möglich, Stützschlauch und/oder Sonde als Einweg-Geräte auszugestalten, während das Griffstück wieder verwendet werden kann.

Das kryochirurgische Instrument kann derart ausgestaltet werden, dass der Überstand des Sondenkopfs in der ausgefahrenen Position mindestens 5, insbesondere 15 mm beträgt. Somit wird eine gute Einsicht auf die Sondenspitze sichergestellt. Der Arzt hat beim Gefriervorgang eine gute Sicht auf den Sondenkopf und dessen Position. Als Überstand wird der Abstand des Sondenkopfs zur Abschlussebene des Distalendes des Stützschlauchs bezeichnet.

Der Stützschlauch kann aus Kunststoff gefertigt sein. Durch das Verwenden von Kunststoff kann eine ausreichende Knickfestigkeit des Stützschlauchs erzielt werden, so dass dieser beim Abrissvorgang nicht einknickt. Des Weiteren hat Kunststoff den Vorteil, dass es sich hierbei um einen thermischen Isolator handelt. Somit wird verhindert, dass das Distalende des Stützschlauchs beim Abkühlen des Sondenkopfs am Gewebe festfriert.

Der Sondenkopf kann aus einem metallischen Werkstoff hergestellt sein.

Bevorzugte Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen:
- - Fig. 1: ein erfindungsgemäßes kryochirurgisches Instrument;
- - Fig. 2: eine Kryosonde;
- - Fig. 3: die Kryosonde aus Fig. 2 mit einem Stützschlauch;
- - Fig. 4: einen Querschnitt durch das kryochirurgische Instrument aus Fig. 1;
- - Fig. 5: das distale Ende der in einem Arbeitskanal eines Endoskops geführten Sonde mit Stützschlauch im Schnitt;
- - Fig. 6 und 7: den erfindungsgemäßen Entnahmevorgang einer Gewebeprobe;
- - Fig. 8: eine mechanische Beschleunigungsvorrichtung;
- - Fig. 9: eine elektromagnetische Beschleunigungsvorrichtung;
- - Fig. 10: eine pneumatische Beschleunigungsvorrichtung;
- - Fig. 11: eine Beschleunigungsvorrichtung mit Elektromotor; und
- - Fig. 12: eine pyrotechnische Beschleunigungsvorrichtung.

In der nachfolgenden Beschreibung werden für gleiche und gleich wirkende Teile dieselben Bezugsziffern verwendet.

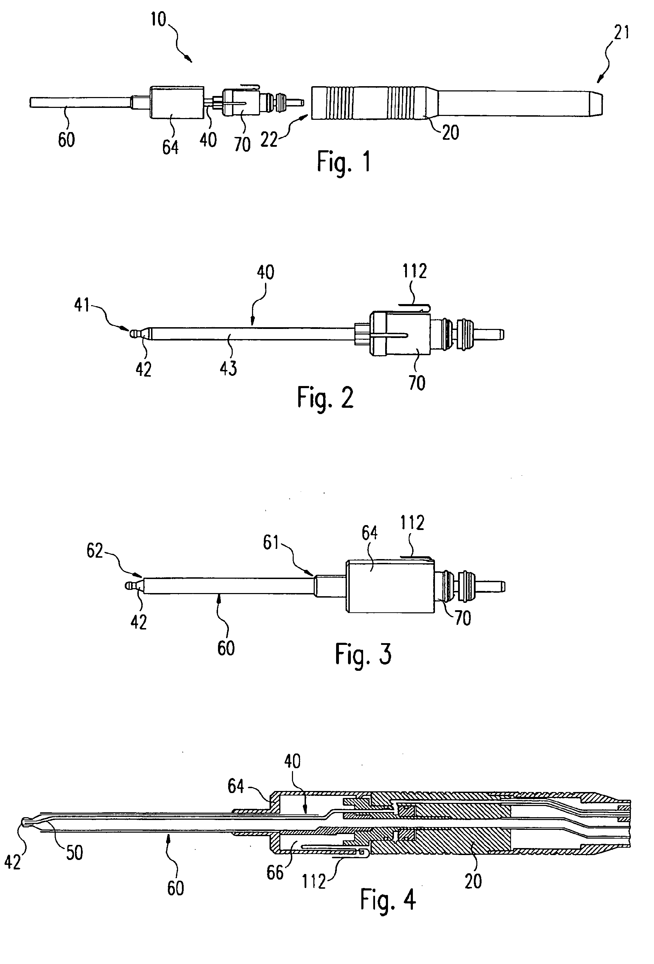

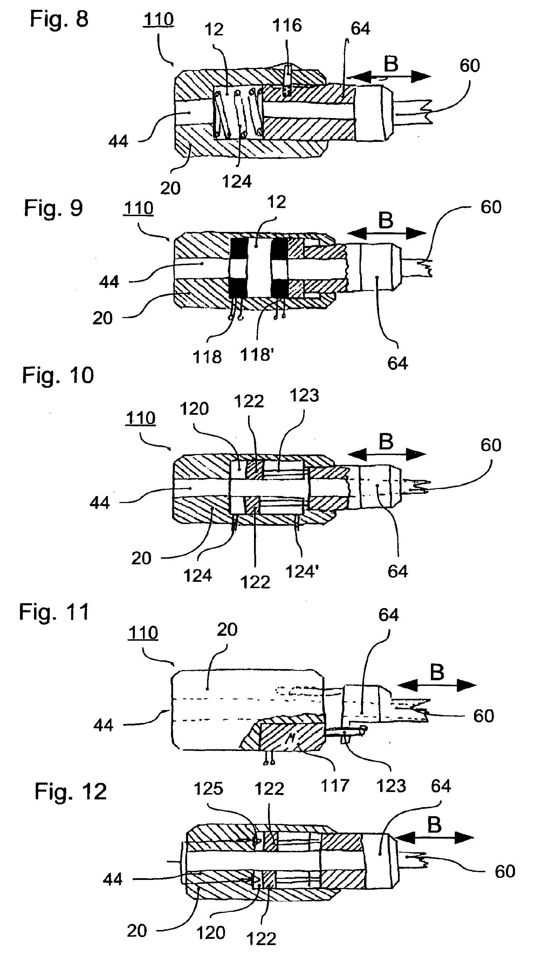

Das erfindungsgemäße kryochirurgische Instrument besteht aus drei wesentlichen Baugruppen. Diese sind in Fig. 1 dargestellt:
- Der Stützschlauch 60 mit Verbindungsstück 64, der als Schutz und zum Herauslösen des Biopsats dient.
   Der Stützschlauch 60 ist mittels Verbindungsstück 64 beweglich an einer Kupplungseinheit 70 fixiert.
- Die Kupplungseinheit 70 mit Sonde 40.
   Die Kupplungseinheit 70, umfassend eine Blattfeder 112, bildet einen Teil der Beschleunigungsvorrichtung 110 (Fig. 8-12).
- Eine Griffeinrichtung 20 zum Halten des kryochirurgischen Instruments 10.

Durch Kupplungen zwischen den einzelnen Baugruppen lassen sich die einzelnen Teile des kryochirurgischen Instruments 10 abnehmen und können nach Bedarf ausgetauscht oder gereinigt werden. Somit ist das System flexibel und für die Handhabung attraktiver, als komplett geschlossene Systeme. Die Griffeinrichtung 20 hat ein proximales Ende 21 und ein distales Ende 22. Die Kupplungseinheit 70 wird am distalen Ende 22 mit der Griffeinrichtung 20 verbunden.

Fig. 2 zeigt die Sonde 40 im Detail. Diese setzt sich aus einem Sondenkopf 42, einem Sondenkörper 43 und der Kupplungseinheit 70 zusammen. Das distale Ende 41 der Sonde 40 bildet also der Sondenkopf 42, der mit dem Sondenkörper 43 verklebt ist. Dieser Sondenkörper 43 ist am proximalen Ende der Sonde 40 mit der Kupplungseinheit 70 verbunden, die eine Blattfeder 112 zum Auslösen einer Beschleunigungseinrichtung 110 (Fig. 8-12) umfasst.

Fig. 3 zeigt die Sonde 40 aus Fig. 2 mit dem Stützschlauch 60. Dieser ist, wie bereits beschrieben, an seinem proximalen Ende 61 mit einem Verbindungsstück 64 verbunden. Das Verbindungsstück 64 hat eine zylindrische Form und lässt sich teilweise über die Kupplungseinheit 70 schieben. Beim Überschieben rastet ein Abschnitt der Blattfeder 112 in eine korrespondierende Öffnung des Verbindungsstücks 64 ein. Im Inneren des Verbindungsstücks 64 befindet sich ein Hohlraum 66 (vgl. Fig. 4), in dem eine Spiralfeder 114 (vgl. Fig. 8) angeordnet ist. Durch das Überziehen des Verbindungsstücks 64 wird diese Spiralfeder 114 gespannt. Bei einer Betätigung der Blattfeder 114 löst sich diese aus der Öffnung am Verbindungsstück 64 und setzt die in der gespannten Spiralfeder 114 gespeicherte potentielle Energie frei. Das Verbindungsstück 64 mit dem Stützschlauch 60 wird hierdurch gegenüber der Kupplungseinheit 70 mit der Sonde 40 in distale Richtung beschleunigt. Wie in Fig. 3 gezeigt, ragt im eingerasteten Zustand der Sondenkopf 42 mehrere Millimeter über das Distalende 62 des Stützschlauchs 60 heraus. Beim Auslösen der vorgespannten Feder 114 schiebt sich der Stützschlauch 60 über den Sondenkopf 42.

Fig. 4 zeigt einen Schnitt entlang der Längsachse des kryochirurgischen Instruments 10. In diesem Schnitt ist der Hohlraum 66, der im eingerasteten Zustand durch das Verbindungsstück 64 und die Kupplungseinheit 70 gebildet wird und sich ringförmig um den Sondenkörper 43 erstreckt, erkennbar. Die Spiralfeder 114 ist in Fig. 4 nicht dargestellt.

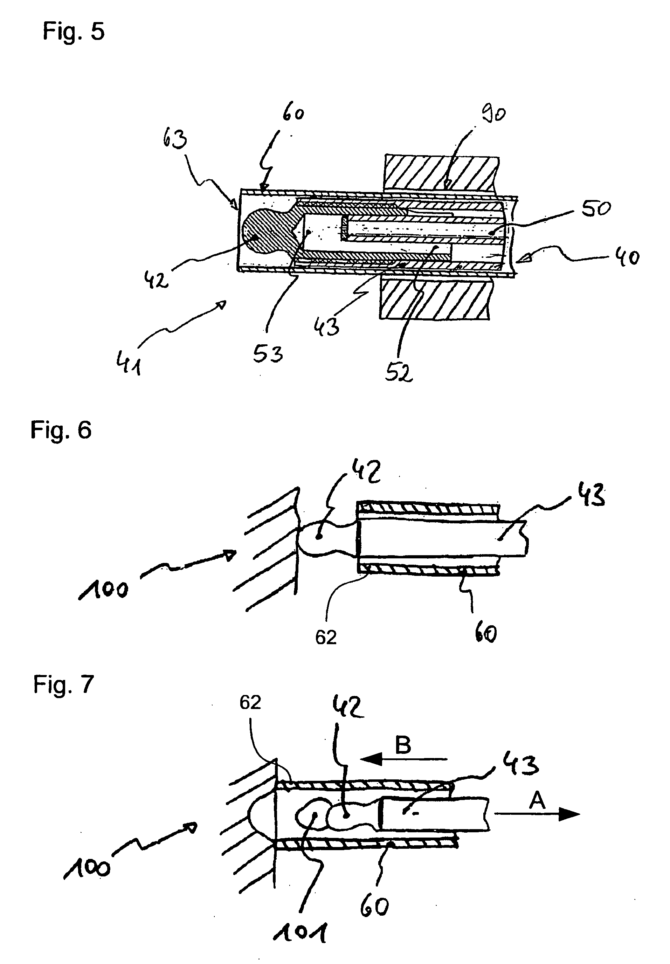

Die Sonde 40 verläuft entlang der Längsachse des kryochirurgischen Instruments durch den Hohlraum 66. Zur Kühlung des Sondenkopfs 42 enthält die Sonde 40 eine Gaszuführleitung 50. Das distale Ende 41 der Sonde 40 ist genauer in der Fig. 5 dargestellt, wobei der Stützschlauch 60 über den Sondenkopf 42 geschoben ist. Die Sonde 40 ist in dem Stützschlauch 60 geführt, wobei mindestens der Sondenkopf 42 in dem Stützschlauch 60 aufnehmbar und aus diesem wieder freigebbar ist. Ferner ist in Fig. 5 ein Arbeitskanal 90 eines Endoskops gezeigt, in welchen die Sonde 40 mit Stützschlauch 60 eingeführt ist. Der Sondenkopf 42 weist am distalen Ende eine im Wesentlichen kugelförmige Ausbildung auf, die einen großflächigen Bereich zur Kontaktierung mit dem Gewebe 100 bereitstellt. Die Form des Sondenkopfes 42 unterstützt die Adhäsion von Gewebe 100 bei der Abkühlung. Das distale Ende 62 des Stützschlauchs 60 hat eine Aufsetzkante 63, die zur Gewebeentnahme auf der Gewebeoberfläche aufsetzt.

Fig. 6 und 7 zeigen die Entnahme der Gewebeprobe 101 von dem zu behandelnden Gewebe 100. In der Fig. 6 überragt der Sondenkopf 42 das distale Ende 62 des Stützschlauchs 60 und liegt auf dem zu behandelnden Gewebe 100 auf. Der Sondenkopf 42 befindet sich also in einer ausgefahrenen Position. Sobald das Gewebe bereichsweise an den Sondenkopf 42 angefroren ist, kann dieser Abschnitt, der schließlich die Gewebeprobe 101 bildet, durch das Gegeneinanderbewegen von Sonde 40 und Stützschlauch 60 von dem umliegenden Gewebe 100 abgetrennt und in den Stützschlauch 60 eingeholt werden. Der Sondenkopf 42 und die Gewebeprobe 101 befinden sich dann im Inneren des Stützschlauchs 60. Diese Position zeigt Fig. 7. Für die erfindungsgemäße Entnahme der Gewebeprobe 101 wird der Stützschlauch 60 durch die Beschleunigungsvorrichtung 110 in distaler Richtung beschleunigt (vgl. Bewegungsrichtung B). Hierbei handelt es sich um eine Relativbewegung zur Sonde 40. Sobald das Distalende 62 des Stützschlauchs 60 auf die Gewebeoberfläche des zu behandelnden Gewebes 100 trifft, induziert dies einen Impuls auf die Sonde 40, umfassend den Sondenkörper 43 und den Sondenkopf 42. Dieser Impuls richtet sich entgegen der Bewegungsrichtung B des Stützschlauchs 60. Somit wird die Sonde 40 in die Bewegungsrichtung A beschleunigt. Die Gewebeprobe 101, die am Sondenkopf 42 fixiert ist, wird aus dem zu behandelnden Gewebe 100 herausgerissen.

Die Fig. 8 bis 12 zeigen zahlreiche Ausgestaltungen der Beschleunigungsvorrichtung 110. Sämtliche beispielhaft dargestellten Beschleunigungsvorrichtungen 110 sind in einen Teil einer Griffeinrichtung 20 integriert. Entlang der Längsachse der Beschleunigungsvorrichtungen 110 verläuft ein Sondenkanal 44, in dem die Sonde 40 (nicht dargestellt) angeordnet ist. Vorzugsweise ist die Sonde 40 mit der Griffeinrichtung 20 verklebt. Das Verbindungsstück 64 lässt sich gegenüber der Griffeinrichtung 20, so wie gegenüber der Sonde 40 in Längsrichtung verschieben (vgl. Bewegungsrichtung B). Fest mit dem Verbindungsstück 64 verbunden ist der Stützschlauch 60. Zu den beispielhaften Ausführungsformen aus den Fig. 8 bis 12 ist noch anzumerken, dass hier entgegen dem Ausführungsbeispiel aus Fig. 1 bis 3 das Verbindungsstück 64 in die Griffeinrichtung hineinragt.

Fig. 8 zeigt ein erstes Ausführungsbeispiel der Beschleunigungsvorrichtung 110, wobei die Beschleunigung mechanisch durch die Spiralfeder 114 gewährleistet wird. Die Spiralfeder 114 ist in dem Hohlraum 66, der durch die Griffeinrichtung 20 und das Verbindungsstück 64 gebildet wird, angeordnet. Die Spiralfeder 114 setzt sowohl an der Griffeinrichtung 20 und dem Verbindungsstück 64 an. Eine Auslösevorrichtung 116 ermöglicht das Einrasten des Verbindungsstücks 64 in einem gespannten Zustand. Bei Bedarf kann die gespeicherte potentielle Energie durch das Betätigen der Auslösevorrichtung 116 freigesetzt werden.

Fig. 9 zeigt eine elektromagnetische Beschleunigungsvorrichtung. Ringförmige Spulen 118, 118' sind in dem Hohlraum 66, den Sondenkanal 44 umgebend angeordnet. Eine erste elektromagnetische Spule 118 ist mit der Griffeinrichtung 20 und eine zweite elektromagnetische Spule 118' mit dem Verbindungsstück 64 verbunden. Die elektromagnetischen Spulen 118, 118' lassen sich derart ansteuern, dass sie sich entweder anziehen oder abstoßen. Hierdurch kann der Stützschlauch 60 in proximale oder distale Richtung bewegt werden.

Fig. 10 zeigt eine pneumatische Beschleunigungsvorrichtung 110. Um den Sondenkanal 44 herum ist ein ringförmiger Zylinder 120 ausgebildet. In diesem Zylinder 120 befindet sich ein entsprechend geformter Kolben 122, der über ein Gestänge 123 mit dem Verbindungsstück 64 verbunden ist. Der Zylinder 120, der sich im Wesentlichen längs zur Längsachse der Beschleunigungsvorrichtung 110 erstreckt, umfasst eine erste Druckluftleitung 124, die proximal in den Kolben 120 mündet, und eine zweite Druckluftleitung 124', die distal in den Kolben 120 mündet. Durch ein Zuführen von Druckluft über die erste Druckluftleitung 124 oder über die zweite Druckluftleitung 124' lässt sich der Kolben 122 heben bzw. senken. Hierdurch wird der Stützschlauch 60 bewegt. Durch eine entsprechende Dosierung der Druckluft lässt sich die Beschleunigung des Stützschlauchs 60 steuern.

Fig. 11 zeigt eine weitere elektromagnetische Beschleunigungsvorrichtung 110. Die Bewegung des Stützschlauchs 60 wird durch einen Motor 117, der über ein Gestänge 123 mit dem Verbindungsstück verbunden ist, gewährleistet.

Fig. 12 zeigt eine pyrotechnische Beschleunigungsvorrichtung 110. Die Beschleunigungsvorrichtung 110 ist ähnlich aufgebaut wie die aus Fig. 10. Sie umfasst einen ringförmigen Zylinder 120 und einen entsprechend ringförmigen Kolben 122, der mit dem Stützschlauch 60 verbunden ist. Im Inneren des Zylinders 120 befindet sich eine pyrotechnische Einheit, die durch eine Zündeinrichtung 125 auslösbar ist. Die durch die chemische Reaktion freigesetzte Kraft wird über Kolben 122 und Gestänge 123 auf das Verbindungsstück 64 übertragen.

### Bezugszeichenliste

- 10: Kryochirurgisches Instrument
- 20: Griffeinrichtung
- 21: proximales Ende der Griffeinrichtung
- 22: distales Ende der Griffeinrichtung
- 40: Sonde
- 41: distales Ende der Sonde
- 42: Sondenkopf
- 43: Sondenkörper
- 44: Sondenkanal
- 50: Gaszuführleitung
- 60: Stützschlauch
- 61: proximales Ende des Stützschlauchs
- 62: distales Ende des Stützschlauchs
- 63: Aufsetzkante
- 64: Verbindungsstück
- 65: Halteeinrichtung
- 66: Hohlraum
- 70: Kupplungseinheit
- 90: Arbeitskanal eines Endoskops
- 100: zu behandelndes Gewebe
- 101: Gewebeprobe
- 110: Beschleunigungsvorrichtung
- 112: Blattfeder
- 114: Spiralfeder
- 116: Auslösevorrichtung
- 117: Motor
- 118, 118': elektromagnetische Spule
- 120: Zylinder
- 122: Kolben
- 123: Gestänge
- 124, 124': Druckluftleitung
- 125: Zündeinrichtung
- A: Bewegungsrichtung der Sonde
- B: Bewegungsrichtung der Stützeinrichtung

## Patentansprüche

1. Kryochirurgisches Instrument zur Gewinnung einer Gewebeprobe (101), umfassend:
- eine Sonde (40) mit einem Sondenkopf (42) der zur Fixierung eines Abschnitts einer Gewebewand durch eine Kühleinrichtung kühlbar ist;
- einen Stützschlauch (60) mit einem Distalende (62), wobei die Sonde (40) in diesem Stützschlauch (60) relativ zu diesem beweglich gelagert ist;
**gekennzeichnet durch**:
eine Beschleunigungsvorrichtung (110) zur Beschleunigung des Stützschlauchs (60) mit einer vorbestimmten Beschleunigungskraft relativ zur Sonde, wobei die Beschleunigungskraft derart gerichtet ist, dass die Sonde (40) aus einer ausgefahrenen Position, in der der Sondenkopf (42) das Distalende (62) des Stützschlauches (60) überragt, im Inneren des Stützschlauches (60) zu liegen kommt, wobei das Distalende (62) des Stützschlauches (60) derart ausgestaltet ist,
dass es nach einer Beschleunigung **durch** die Beschleunigungsvorrichtung (110) bei einer Kontaktierung der Gewebewand eine Abrisskraft so induziert, dass **durch** die Sonde (40) ein Abschnitt der Gewebewand herausgerissen wird.

2. Kryochirurgisches Instrument nach Anspruch 1,
**dadurch gekennzeichnet, dass**
der Sondenkopf (42) im Wesentlichen einen Planarabschnitt zur Kontaktierung mit der Gewebewand umfasst.

3. Kryochirurgisches Instrument nach einem der vorhergehenden Ansprüche,
**dadurch gekennzeichnet, dass**
die Beschleunigungsvorrichtung (110) eine pneumatische und/oder eine elektromagnetische und/oder eine mechanische und/oder pyrotechnische Beschleunigungsvorrichtung (110) umfasst.

4. Kryochirurgisches Instrument nach einem der vorhergehenden Ansprüche,
**dadurch gekennzeichnet, dass**
die Beschleunigungsvorrichtung (110) eine Feder (114) zur Speicherung einer vorbestimmten Menge an potentieller Energie umfasst.

5. Kryochirurgisches Instrument nach einem der vorhergehenden Ansprüche,
**dadurch gekennzeichnet, dass**
die Beschleunigungsvorrichtung (110) ein Verbindungsstück (64) umfasst, das mit dem Stützschlauch (60) in Wirkverbindung steht und mittelbar oder unmittelbar an die Sonde (40) angekuppelt ist, wobei das Verbindungsstück (64) ein Spannelement umfasst, das eine vorbestimmte Menge an potentieller Energie speichert.

6. Kryochirurgisches Instrument nach Anspruch 5,
**dadurch gekennzeichnet, dass**
die Beschleunigungsvorrichtung (110) eine Auslösevorrichtung (116) umfasst, die die potentielle Energie freisetzt.

7. Kryochirurgisches Instrument nach einem der vorhergehenden Ansprüche,
**gekennzeichnet durch**
ein Griffstück, wobei die Sonde (40) abnehmbar an das Griffstück angekuppelt ist.

8. Kryochirurgisches Instrument nach einem der vorhergehenden Ansprüche,
**dadurch gekennzeichnet, dass**
der Stützschlauch (60) und die Sonde (40) derart bemessen sind, dass der Überstand des Sondenkopfs (42) in der ausgefahrenen Position mindestens 5 Millimeter (mm) beträgt.

9. Kryochirurgisches Instrument nach einem der vorhergehenden Ansprüche,
**dadurch gekennzeichnet, dass**
der Stützschlauch (60) aus Kunststoff ist.

10. Kryochirurgisches Instrument nach einem der vorhergehenden Ansprüche,
**dadurch gekennzeichnet, dass**
der Sondenkopf (42) aus einem metallischen Werkstoff ist.

## Claims

1. Cryosurgical instrument for obtaining a tissue sample (101), comprising:
- a probe (40) having a probe head (42) which, for fixing a portion of a tissue wall, is coolable by a cooling device;
- a support tube (60) having a distal end (62), the probe (40) being mounted in the support tube (60) so as to be movable relative thereto;
**characterized by**:
an acceleration device (110) for accelerating the support tube (60) with a predetermined accelerating force relative to the probe, the accelerating force being directed in such a way that the probe (40), starting from an extended position in which the probe head (42) projects beyond the distal end (62) of the support tube (60), comes to rest in the interior of the support tube (60), the distal end (62) of the support tube (60) being configured in such a way that,
after acceleration by the acceleration device (110), on making contact with the tissue wall a tear-off force is so induced that a portion of the tissue wall is torn out by the probe (40).

2. Cryosurgical instrument according to claim 1,
**characterized in that**
the probe head (42) comprises a substantially planar portion for making contact with the tissue wall.

3. Cryosurgical instrument according to either one of the preceding claims,
**characterized in that**
the acceleration device (110) comprises a pneumatic and/or an electromagnetic and/or a mechanical and/or pyrotechnic acceleration device (110).

4. Cryosurgical instrument according to any one of the preceding claims,
**characterized in that**
the acceleration device (110) comprises a spring (114) for storing a predetermined amount of potential energy.

5. Cryosurgical instrument according to any one of the preceding claims,
**characterized in that**
the acceleration device (110) comprises a connecting piece (64) which is in operative connection with the support tube (60) and is indirectly or directly coupled to the probe (40), the connecting piece (64) comprising a tensionable element which stores a predetermined amount of potential energy.

6. Cryosurgical instrument according to claim 5,
**characterized in that**
the acceleration device (110) comprises a trigger device (116) which releases the potential energy.

7. Cryosurgical instrument according to any one of the preceding claims,
**characterized by**
a grip piece, the probe (40) being detachably coupled to the grip piece.

8. Cryosurgical instrument according to any one of the preceding claims,
**characterized in that**
the support tube (60) and the probe (40) are so dimensioned that the projecting length of the probe head (42) in the extended position is at least 5 millimetres (mm).

9. Cryosurgical instrument according to any one of the preceding claims,
**characterized in that**
the support tube (60) is made of plastics material.

10. Cryosurgical instrument according to any one of the preceding claims,
**characterized in that**
the probe head (42) is made of a metallic material.

## Revendications

1. Instrument de cryochirurgie pour le prélèvement d'un échantillon de tissu (101), comportant :
- une sonde (40) avec une tête (42) qui peut être refroidie par un dispositif de refroidissement pour l'immobilisation d'une partie d'une paroi tissulaire ;
- un tuyau flexible de support (60) avec une extrémité distale (62), la sonde (40) étant logée dans ce tuyau flexible de support (60) de manière mobile par rapport à ce dernier ;
**caractérisé par**
un dispositif d'accélération (110) pour accélérer le tuyau flexible de support (60) avec une force d'accélération prédéterminée par rapport à la sonde, la force d'accélération étant dirigée de telle sorte que la sonde (40) est amenée hors d'une position extraite, dans laquelle la tête (42) de la sonde s'avance au-delà de l'extrémité distale (62) du tuyau flexible de support (60), et vient en appui à l'intérieur du tuyau flexible de support (60), l'extrémité distale (62) du tuyau flexible de support (60) étant configurée de telle sorte que, après une accélération au moyen du dispositif d'accélération (110), en entrant en contact avec la paroi tissulaire, elle induit une force d'arrachage telle qu'une partie de la paroi tissulaire est arrachée au moyen de la sonde (40).

2. Instrument de cryochirurgie selon la revendication 1, **caractérisé en ce que** la tête (42) de la sonde comporte pour l'essentiel une partie plane destinée à venir en contact avec la paroi tissulaire.

3. Instrument de cryochirurgie selon l'une quelconque des revendications précédentes, **caractérisé en ce que** le dispositif d'accélération (110) comporte un dispositif d'accélération (110) pneumatique et/ou électromagnétique et/ou mécanique et/ou pyrotechnique.

4. Instrument de cryochirurgie selon l'une quelconque des revendications précédentes, **caractérisé en ce que** le dispositif d'accélération (110) comporte un ressort (114) destiné à stocker une quantité prédéterminée d'énergie potentielle.

5. Instrument de cryochirurgie selon l'une quelconque des revendications précédentes, **caractérisé en ce que** le dispositif d'accélération (110) comporte une pièce de liaison (64) qui est en liaison active avec le tuyau flexible de support (60) et qui est couplée indirectement ou directement à la sonde (40), la pièce de liaison (64) comportant un élément de serrage qui stocke une quantité prédéterminée d'énergie potentielle.

6. Instrument de cryochirurgie selon la revendication 5, **caractérisé en ce que** le dispositif d'accélération (110) comporte un dispositif de déclenchement (116) qui libère l'énergie potentielle.

7. Instrument de cryochirurgie selon l'une quelconque des revendications précédentes, **caractérisé par** un manche, la sonde (40) étant couplée au manche de manière amovible.

8. Instrument de cryochirurgie selon l'une quelconque des revendications précédentes, **caractérisé en ce que** le tuyau flexible de support (60) et la sonde (40) sont dimensionnés de telle sorte que le porte-à-faux de la tête (42) de la sonde dans la position extraite mesure au moins 5 millimètres (mm).

9. Instrument de cryochirurgie selon l'une quelconque des revendications précédentes, **caractérisé en ce que** le tuyau flexible de support (60) est en matière plastique.

10. Instrument de cryochirurgie selon l'une quelconque des revendications précédentes, **caractérisé en ce que** la tête (42) de la sonde est réalisée dans un matériau métallique.
